# EUROPEAN PATENT SPECIFICATION

(11) **EP 0 923 571 B1**
(45) Date of publication and mention of the grant of the patent: **18.06.2003**
(21) Application number: 97936183.9
(22) Date of filing: 29.07.1997
(51) Int. Cl.: C07D 401/12, A01N 43/56, C07D 213/69, A01N 43/40

(54) **TRISUBSTITUTED PYRIDINE COMPOUNDS FOR HERBICIDAL USE**
TRISUBSTITIERTE PYRIDINVERBINDUNGEN ZUR ANWENDUNG ALS HERBIZIDE
COMPOSES DE PYRIDINE TRISUBSTITUEE UTILISES EN TANT QU'HERBICIDES

(30) Priority: 30.07.1996 US 688570
(43) Date of publication of application: 23.06.1999
(73) Proprietor: BASF AKTIENGESELLSCHAFT, 67056 Ludwigshafen (DE)
(72) Inventor: BALTRUSCHAT, Helmut, Siegfried, D-55444 Schweppenhausen (DE); KLEEMANN, Axel, D-63454 Hanau (DE); MAIER, Thomas, D-55127 Mainz (DE); SCHEIBLICH, Stefan, D-55128 Mainz (DE); PONT, Joseph, Luke, Newtown PA 18940 (US); HOELLMUELLER, Thomas, D-55435 Gau-Algesheim (DE)
(74) Representative: Langfinger, Klaus Dieter, Dr.
(86) International application number: US9712947
(87) International publication number: WO98004548

(56) References cited:
- EP-A- 0 572 093
- WO-A-94/08991
- WO-A-94/22833

## Description

### BACKGROUND OF THE INVENTION

Selective herbicides play an important role in agriculture and related fields. Certain pyridine derivatives have been suggested as active ingredients for such herbicides. For example, WO 94/22833 describes compounds of formula Q 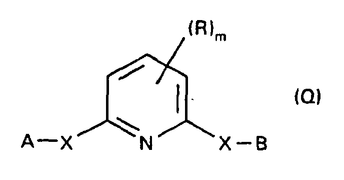 wherein
A represents an optionally substituted 5- or 6-membered nitrogen containing heteroaromatic group, B represents an optionally substituted 5- or 6-membered cyclic hydrocarbon, alkyl, alkenyl, alkynyl, aryl or aralkyl group, or one of the meanings of A, R represents a halogen atom or an alkyl, haloalkyl, alkoxy, alkylthio or dialkylamino group, m represents 0, 1 or 2 and X represents an oxygen or sulphur atom.

It has now surprisingly been found that within the huge number of compounds covered by formula Q in a general manner there are certain compounds of excellent selective herbicidal activity, which have not yet been described. These compounds and their use form the subject matter of the present invention.

### Summary of the invention

The present invention provides the novel compounds of formula IA 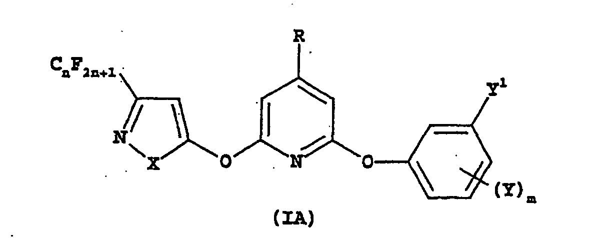 in which
- R: represents a methyl, ethyl, methoxy, ethoxy, methylthio or ethylthio group or a fluorine, chlorine or bromine atom;
- X: represents -N(CH₃)-;
- Y¹: represents a fluorine or chlorine atom or a methyl, trifluoromethyl or nitro group;
- Y: each independently represents a fluorine or chlorine atom or a methyl, trifluoromethyl or nitro group;
- m: is an integer from 0 to 2; and
- n: is 1 or 2;
with the proviso that m is 1 or 2, if Y¹ represents a fluorine atom;
and the agronomically acceptable salts thereof;
and compounds of formula IB, 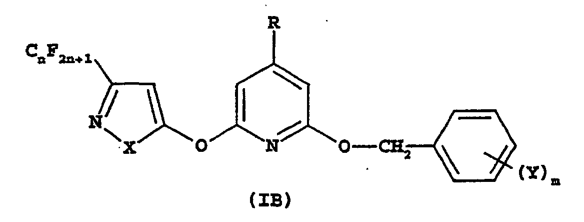 in which
- R: represents a methyl, ethyl, methoxy, ethoxy, methylthio or ethylthio group or a fluorine, chlorine or bromine atom;
- X: represents -N(CH₃)-;
- Y: each independently represents a fluorine or chlorine atom or a methyl, trifluoromethyl or nitro group;
- m: is an integer from 1 to 3; and
- n: is 1 or 2;
and the agronomically acceptable salts thereof.

The compounds of formulae IA and IB show an unexpectedly superior activity and/or selectivity compared with the compounds described in WO 94/22833.

It is an object of the present invention to provide herbicidal compounds having superior activity or selectivity.

It is another object of the invention to provide methods for controlling undesired plant growth by contacting said plants with a herbicidally effective amount of the compounds according to the h-vention.

It is yet another object of the invention to provide selective herbicidal compositions containing the compounds of this invention as active ingredients.

These and other objects and features of the invention will become more apparent from the detailed description set forth hereinbelow, and from the appended claims.

### DETAILED DESCRIPTION OF THE INVENTION

It has surprisingly been found that the trisubstituted pyridines of the formula IA and IB 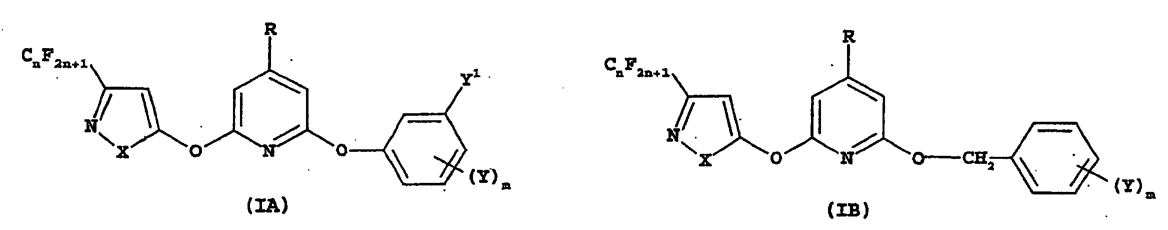 in which n, X, R, Y, Y¹ and m have the meaning given unexpectedly show an excellent herbicidal activity against Alopecurus myosuorides in cereal. The novel compounds of formulae IA and IB additionally exhibit an excellent herbicidal activity combined with high selectivity, e.g. in soybeans, maize, and/or barley.

In a preferred embodiment of the invention R is methyl or methoxy; and (Y)ₘ each independently represents hydrogen or fluorine atoms.

Particularly preferred are those compounds of formula I A wherein n is 1; the group 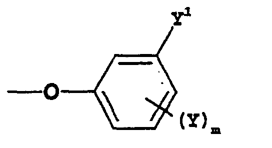 is chosen from 3,4-difluorophenyl, 3,5-difluorophenyl, 3-chloro-4-fluorophenyl, 3-trifluoromethyl-4-fluorophenyl and 3-trifluoromethyl-4-chlorophenyl; and R is methyl or methoxy.

Also particularly preferred are those compounds of formula IB wherein n is 1; the group 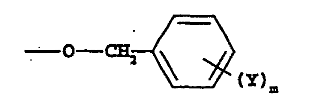 is chosen from fluorobenzyl (e.g. 4-fluorobenzyl), methylbenzyl (e.g. 2-methylbenzyl), chlorobenzyl, difluorobenzyl, dichlorobenzyl, difluorobenzyl (e.g. 2,4-difluorobenzyl, 3,4-difluorobenzyl), fluoro-trifluoromethylbenzyl or chloro-trifluoromethylbenzyl; and R is methyl or methoxy.
Most preferred are the compounds of formula IA and IB selected from the group consisting of
2-(1-methyl-3-trifluoromethylpyrazol-5-yloxy)-6-(2-methylbenzyloxy)-4-methoxypyridine;
2-(1-methyl-3-trifluoromethylpyrazol-5-yloxy)-6-(2,4-difluorobenzyloxy)-4-methoxypyridine;
2-(1-methyl-3-trifluoromethylpyrazol-5-yloxy)-6-(3,4-difluorobenzyloxy)-4-methoxypyridine;
2-(1-methyl-3-trifluoromethylpyrazol-5-yloxy)-6-(4-fluorobenzyloxy)-4-methylpyridine;
2-(1-methyl-3-trifluoromethylpyrazol-5-yloxy)-6-(3,4-difluorophenoxy)-4-methylpyridine;
2-(1-methyl-3-trifluoromethylpyrazol-5-yloxy)-6-(3-chloro-4-fluorophenoxy)-4-methylpyridine;
2-(1-methyl-3-trifluoromethylpyrazol-5-yloxy)-6-(4-fluoro-3-trifluoromethylphenoxy)-4-methylpyridine;
2-(1-methyl-3-trifluoromethylpyrazol-5-yloxy)-6-(4-chloro-3-trifluoromethylphenoxy)-4-methylpyridine;
2-(1-methyl-3-trifluoromethylpyrazol-5-yloxy)-6-(3-trifluoromethylphenoxy)-4-methylpyridine; and
2-(1-methyl-3-trifluoromethylpyrazol-5-yloxy)-6-(3,5-difluorophenoxy)-4-methylpyridine.

The compounds of this invention can be prepared according to known methods, e.g., as described in WO 94/22833. Examples of suitable processes for making compounds of the present invention are as follows:
( A) A pyridine of formula II: 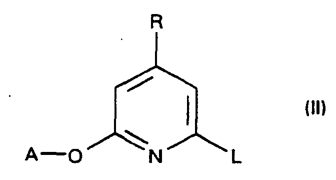 wherein L denotes a leaving group;
   A represents a group of formula 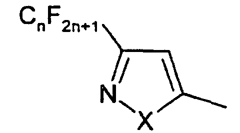 in which n and X have the meaning given, and R is defined as above, is reacted with an equimolar amount of a compound of formula III:

   H-O-B (III),

   wherein B represents a group of the formulae 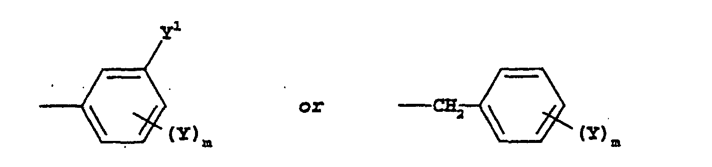 in which Y, Y¹ and m are as defined above;
   or a metal salt of III under basic conditions.
   Leaving groups L; suitably include, e.g., alkyl- or arylsulfonyl groups, alkyl- or arylsulfonyloxy groups, perfluoroalkylsulfonyl or perfluorosulfonyloxy groups, nitro, and halogen (especially fluorine, chlorine and bromine).
(B) A pyridine of formula IV: 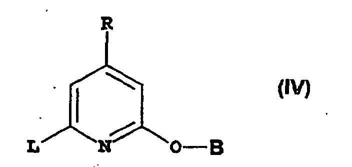 wherein L denotes a leaving group, B and R are defined as above, is reacted with an equimolar amount of a compound of formula V:

   A-O-H (V)

   wherein A is as defined above, or a metal salt of V under basic conditions.
(C) A symmetrically substituted pyridine of formula VI: 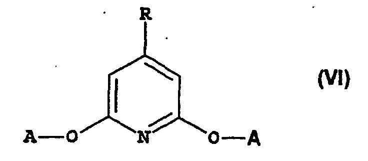 wherein A and R are defined as above, is reacted with an a molar equivalent of a compound III or a metal salt thereof under basic conditions.
(D) A pyridine derivative of formula VII: 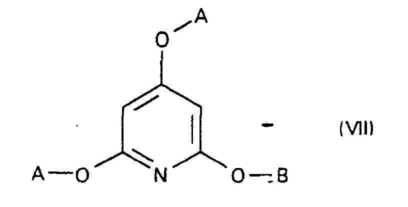 wherein A and B are as defined above, is reacted with an equimolar amount or an excess of a compound of formula VIII:

   C₁₋₂alkyl-X-H (VIII)

   wherein X is a oxygen or sulphur atom, or a metal salt of VIII under basic conditions.
(E) A compound of formula VII is reacted with an equimolar amount or an excess of ammonia to give the corresponding amino compound of formula IX: 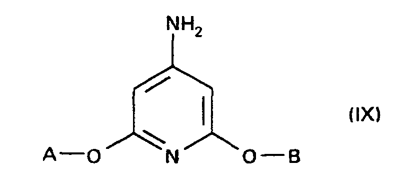
wherein the amino group is diazotated and the resulting intermediate converted into the corresponding fluoro, chloro or bromo compound via a standard halogenation reaction.

The above reactions A to D are conveniently carried out in an organic solvent at elevated temperature. Generally any polar organic solvent is suitable, e.g., dimethylformamide, tetrahydrofurane, sulfolane or pyridines. The presence of a copper salt can be useful. The hydroxy compound of formula III or V is preferably used in form of a metal salt, suitably a sodium or potassium salt, which is conveniently generated by reaction of the hydroxy compound with a basic compound such as a alkali metal hydroxide or carbonate or hydride.

In compounds II, IV and VI R preferably denotes a methyl, ethyl, methoxy, ethoxy, methylthio or ethylthio group.

Compounds of formula IA and IB, wherein R represents a fluorine, chlorine or bromine atom, are suitably prepared via process E. Compound IX is diazotated with e.g., NaNO₂ or alkylONO in the presence of an acid, preferably a halogen containing acid (HCI, HF, HBF₄) or followed by treatment with NOBF₄, in a suitable solvent like pyridine, water, acetonitrile or mixtures of solvents or without a solvent (see Fukuhara Tsuyoshi et al., J.Fluor.Chem. 38, (1988) 435-438; D.J. Milner, Synth. Commun. 22, (1992), 73-82; P. Rocca et al., Tetrahedron Lett. 34, 18, (1993), 2937-2940; S.C.Clayton et al., 34, 46, (1993), 7493-7496; C.F.Allen et al., Org. Synth..III, (1955), 136).

The leaving groups L are preferably fluorine, chlorine or bromine atoms, or SO₂-alkyl, SO₂-phenyl, OSO₂-alkyl, OSO₂-phenyl, OSO₂CF₃ or nitro groups.

Starting from the 2,6-halopyridine of formula X: 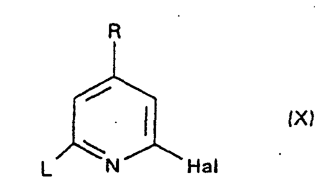 wherein Hal denotes a halogen atom, but can also be replaced by another leaving group, R is defined as above, but preferably represents a methyl, ethyl, methoxy, ethoxy, methylthio or ethylthio group, the reaction with at most an equimolar amount of III or V, in the presence of a base or in the form of a metal salt, leads to IV or II (wherein L is a halogen atom) respectively. Compounds of formula VI can be prepared by reacting X with at least a twofold molar excess of a metal salt of V.

The compound of formula IV can alternatively be prepared starting from a pyridone of formula XI: 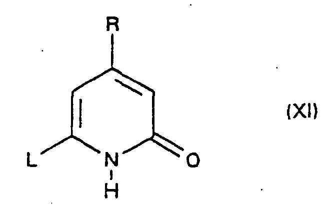 wherein L and R are defined as above.This compound is first converted into the corresponding pyridoxylate by reacting the pyridone with an alkali metal hydroxide. The pyridoxylate is then reacted with a halide of formula XII:

Hal - B (XII)

wherein B and Hal are defined as above.

Compounds of formula VI wherein R is an alkoxy or alkylthio group can be obtained from compounds of formula XIII: 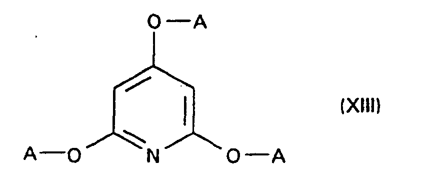 (A is defined as above)
which in turn can be prepared from 2,4,6-trifluoropyridine and a metal salt of V under basic conditions.

The following acids are suitable for the preparation of the agronomically acceptable salts of the compounds of formula I: hydrohalides like hydrochloric or hydrobromic acid, phosphoric acid, nitric acid, sulfuric acid, mono- or bifunctional carboxylic acids and hydroxycarboxylic acids like acetic acid, maleic acid, succinic acid, fumaric acid, citric acid, salicylic acid, sorbic acid or lactic acid and sulfonic acids like p-toluenesulfonic acid or naphthalene-1,5-diyl-disulfonic acid. The agronomically acceptable salts of the compounds of formula I are prepared according to conventional salt formation procedures, for example by dilution of a compound of formula I in a suitable organic solvent, addition of an acid and isolation of the salt formed by, for example, filtration and optional purification by washing with an inert solvent.

The present invention also extends to herbicidal compositions which comprise a compound of formula I and at least one carrier.

Preferably, at least one surface-active agent is also in the composition of the present invention.

A carrier in a composition according to the invention is any material with which the active ingredient may be formulated either to facilitate application to the locus to be treated, which locus may be a plant, seed or soil. or to facilitate storage, transport or handling. A carrier may be a solid or a liquid, including a material which is normally gaseous but which has been compressed to form a liquid. Any of the carriers normally used in formulating herbicidal compositions may be used. Preferably compositions according to the invention contain 0.5 to 95% by weight of active ingredient.

Suitable solid carriers include natural and synthetic clays and silicates, for example natural silicates such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumaron resins, polyvinyl chloride, and styrene polymers and copolymers; solid polychlorophenols; bitumen; waxes; and solid fertilizers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosene and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus, preferably at least one material in a composition according to the invention is a surface active agent. For example, the composition may contain at least two carriers, at least one of which is a surface-active agent.

A surface-active agent may be an emulsifying agent, a dispersing agent or a wetting agent; it may be non-ionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitol, sucrose or pentaerythrol; condensates of these with ethyfene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or earth alkali metal salts, preferably sodium salts; or sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The herbicidal composition of the invention may also contain other active ingredients, for example, compounds possessing insecticidal or fungicidal properties, or other herbicides.

A formulation containing a compound according to the invention, for example, can consist of 100 g of active ingredient (compound of formula I), 30 g of dispersing agent, 3 g of antifoaming agent, 2 g of structure agent, 50 g of anti-freezing agent, 0.5 g of a biocidal agent and sufficient water to make 1000 ml. Prior to use it is diluted with water to give the desired concentration of active ingredient.

The active ingredients according to the invention can be employed alone or as formulations in combination with conventional herbicides. Such combinations of at least two herbicides can be included in the formulation or also added in a suitable form with the preparation of the tank mix. For such mixtures at least one of the following known herbicides can be used:
ametrydione, metabenzthiazuron, metamitron, metribuzin, 2,4-D, 2,4-DB, 2,4-DP, alachlor, alloxydim, asulam, atrazin, bensulfuron, bentazon, bifenox, bromoxynil, butachlor, chloridazon, chlorimuron, chlorpropham, chlorsulfuron, chlortoluron, cinmethylin, clopyralid, cyanazin, cycloate, cycloxydim, dichlobenil, diclofop, eptame, ethiozin, fenoxaprop, fluazifop, fluometuron, fluridone, fluroxypyr, fomesafen, glyphosate, haloxyfop, hexazinone, imazamethabenz, imazapyr, imazaquin, imazethapyr, ioxynil, isoproturon, lactofen, MCPA, MCPP, mefenacet, metazachlor, metolachlor, metsulfuron, molinate, norflurazon, oryzalin, oxyfluorfen, pendimethalin, picloram, pretilachlor, propachlor, pyridate, quizalofopethyl, sethoxydim, simetryne, terbutryne, thiobencarb, triallate, trifluralin, diflufenican, propanil, triclopyr, dicamba, desmedipham, acetochlor, fluoroglycofen, halosafen, tralkoxydim, amidosulfuron, cinosulfuron, nicosulfuron, pyrazosulfuron, thiameturon, thifensulfuron, triasulfuron, oxasulfuron, azimsulfuron, tribenuron, esprocarb, prosulfocarb, terbutylazin, benfuresate, clomazone, di-methazone, dithiopyr, isoxaben, quinchlorac, qinmerac, sulfosate, cyclosulfamuron, imazamox, imazamethapyr, flamprop-M-methyl, flamprop-M-isopropyl, picolinafen, fluthiamid, isoxaflutole, flurtamone, daimuron, bromobutide, methyldimron, dimethenamid, sulcotrione, sulfentrazone, oxadiargyl, acifluorfen, cafenstrole, carfentrazone, diuron, glufosinate.

Mixtures with other active ingredients like fungicides, insecticides, acaricides, and nematicides are possible.

The invention further comprises a method for controlling undesired plants by contacting said plants with an effective amount of a compound of formula I. The amount of active compound needed for such treatment depends to some extent on the compound and the formulation used, the type of undesired plants and the climatic conditions. As a rule, the amount of active compound is 0.005 to 1 kg/ha, preferably 0.01 to 0.5 kg/ha.

For a more clear understanding of the invention, specific examples are set forth below. These examples are merely illustrations and are not to be understood as limiting the scope and underlying principles of the invention in any way. Various modifications of the invention in addition to those shown and described herein will become apparent to those skilled in the art from the following examples and foregoing description. Such modifications are also intended to fall within the scope of the appended claims.

### Example 1

### 2-(1-Methyl-3-trifluoromethylpyrazol-5-yloxy)-6-(3,4-dichlorophenyloxy)-4-methylpyridine

A mixture of 1.5g (5mmol) 2-(1-methyl-3-trifluoromethylpyrazol-5-yloxy)-6-bromo-4-methylpyridine, 1g (6mmol) 3,4-dichlorophenol and 1g (7mmol) potassium carbonate in 5ml sulfolane is heated to 170°C for 12 hours. After cooling, the reaction mixture is diluted with 10ml of a solvent mixture of hexane/ethyl acetate (volume ratio of 1/1) and filtered through a short silica gel column, using the same solvent mixture as an eluent. The filtrate is washed with water and the organic layer dried with anhydrous magnesium sulfate. The solvent is removed in vacuo and the residue purified by a flash silica gel column chromatography using hexane/ethyl acetate in a ratio of 8/2. 0.9g (43% yield) of the title compound are obtained as a white solid of melting point 88°C.

### Example 2

### 2-(1-Methyl-3-trifluoromethylpyrazol-5-yloxy)-6-(2,5-difluorobenzyloxy)-4-methylpyridine

A stirred mixture of 3.2g (7.5mmol) 2,6-Bis-(1-methyl-3-trifluoromethylpyrazol-5-yloxy)-4-methylpyridine, 0.33g (8.25mmol) of sodium hydride (60% dispersion in mineral oil) and 1.1g (8.25mmol) potassium carbonate in 10ml DMF is heated to 80°C. A solution of 0.92ml (8.25mmol) 2,5-difluorobenzyl alcohol in 5ml DMF is added over a period of 20 minutes. The reaction mixture is left overnight. After cooling, the reaction mixture is diluted with 10ml of a solvent mixture of hexane/ethyl acetate (volume ratio of 1/1) and filtered through a short silica gel column using the same solvent mixture as an eluent. The filtrate is washed with water and the organic layer dried with anhydrous magnesium sulfate. The solvent is removed in vacuo and the residue purified by a flash silica gel column chromatography using hexane/ethyl acetate in a ratio of 9/1. The title compound is obtained as a pale yellow solid 2.6g (87% yield) of melting point 67°C.

### Example 3

### 3A 2,4,6-Tris-(1-methyl-3-trifluoromethylpyrazol-5-yloxy)pyridine

A mixture of 4.8 g (36 mmol) 2,4,6-trifluoropyridine, 19.8 g (119 mmol) 5-hydroxy-1-methyl-3-trifluoromethylpyrazole and 18.1 g (131 mmol) potassium carbonate in 25 ml anhydrous sulfolane is stirred and heated to 80°C over a period of 3 days. After cooling, the mixture is diluted with pentanelethyl acetate (1/1) and filtered through a bed of silica gel. The filtrate is washed 10 times with water and the organic layer is dried over magnesium sulfate. After removal of the solvents, the residue is washed with isopropyl ether and 19.1 g (93 % yield) colorless crystals of melting point 130 °C are obtained.

### 3B 2,6-Bis-(1-methyl-3-trifluoromethylpyrazol-5-yloxy)-4-methoxypyridine

A 3 ml aliquot of a 25 % solution of potassium methylate (10 mmol) in dry methanol is added to a solution of 5.7 g (10 mmol) 3A in 20 ml anhydrous methanol. After 2 hours at ambient temperature, the reaction mixture is heated to reflux. The solvent is removed under reduced pressure and ethyl acetate is added to the residue. The mixture is washed with a 2 N sodium hydroxide solution. After drying and filtration of the organic layer, the solvents are removed and the residue is washed with diisopropyl ether and pentane. Colorless crystals (1.9 g, 43 % yield) of melting point 107°C are obtained.

### 3C 4-Methoxy-2-(1-methyl-3-trifluoromethylpyrazol-5-yloxy)-6-(2-methylbenzyloxy)pyridine

A 0.1 g quantity of sodium hydride (60 %, 2.5 mmol) is added to a solution of 2-methylbenzyl alcohol (0.3 g, 2.5 mmol) in sulfolane (5 ml) at 50 °C. After 1 hour at 50 °C, 1 g of 3B is added to the reaction mixture. The mixture is heated to 90 °C for 2 hours and then to 120 °C for 3 hours. After cooling, the reaction mixture is diluted with pentane/ethyl acetate (1/1) and filtered through a bed of silica gel. The filtrate is washed 6 times with water. The organic layer is dried with anhydrous magnesium sulfate, filtered, and evaporated in vacuo. Purification by flash chromatography (silica gel, pentane/ethyl acetate 1/1 v/v) gives the title compound (0.3 g, 33 % yield) of melting point 78 °C.

Following procedures analogous to the Examples 1-3 above, or as explained in the foregoing general description, further compounds according to the invention can be prepared; details are given below in Tables 1 to 4.

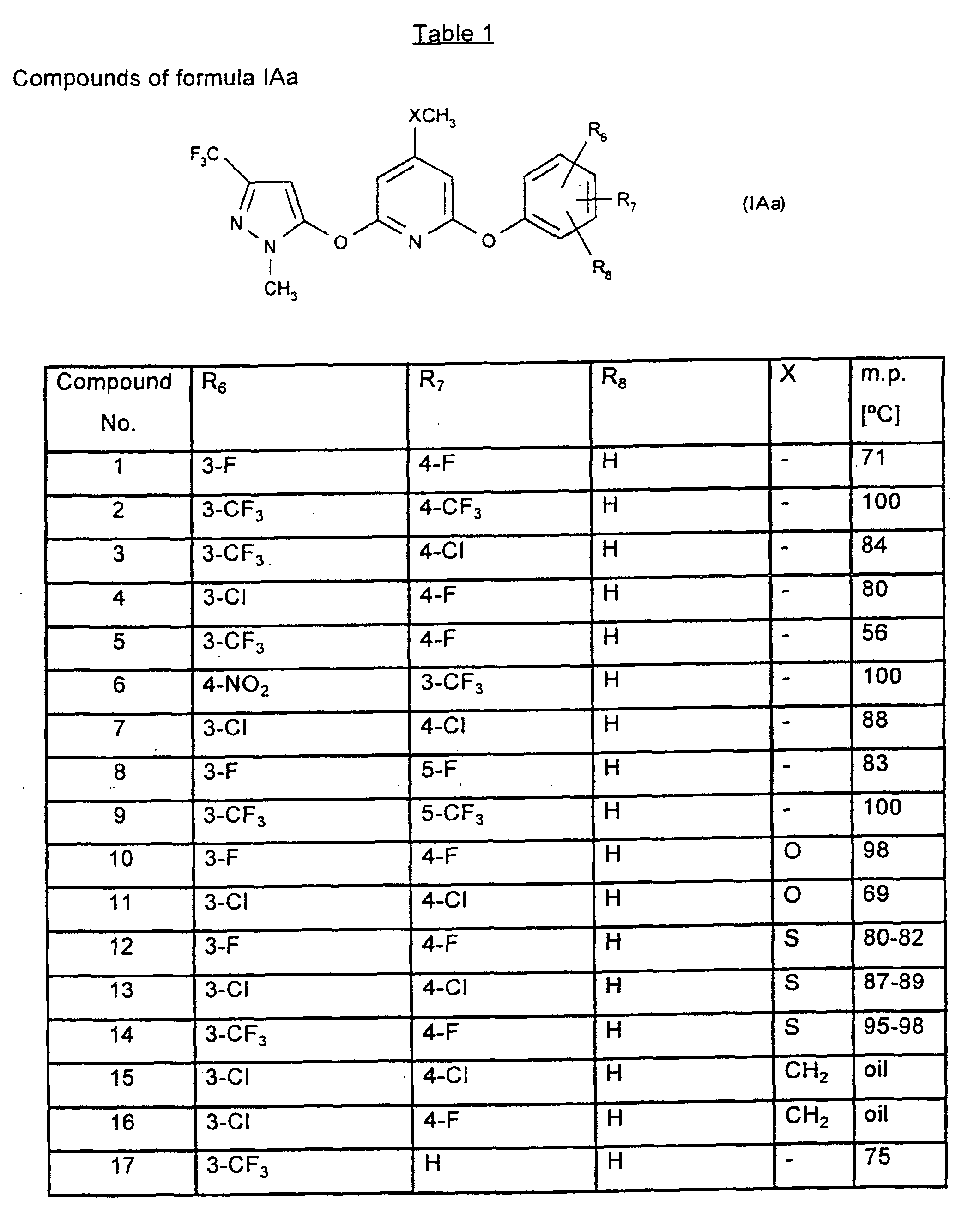

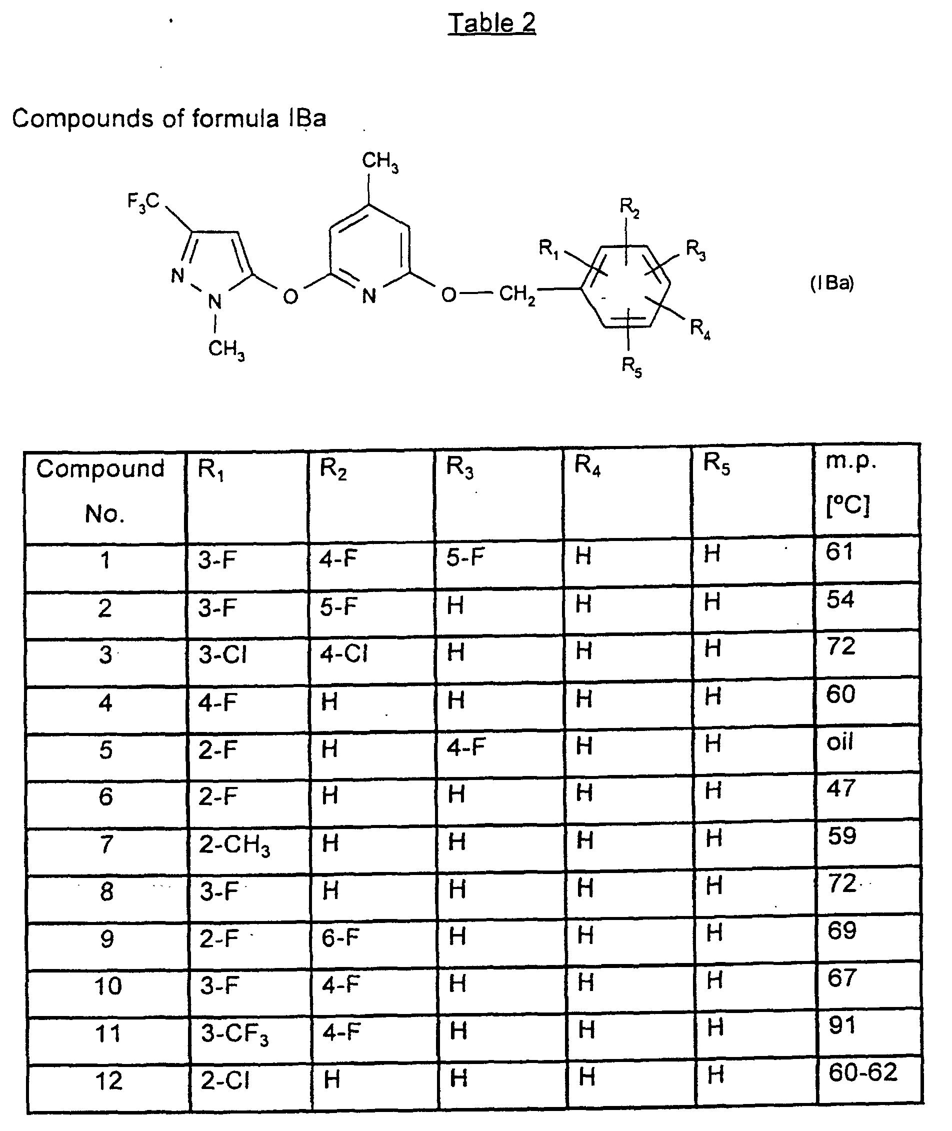

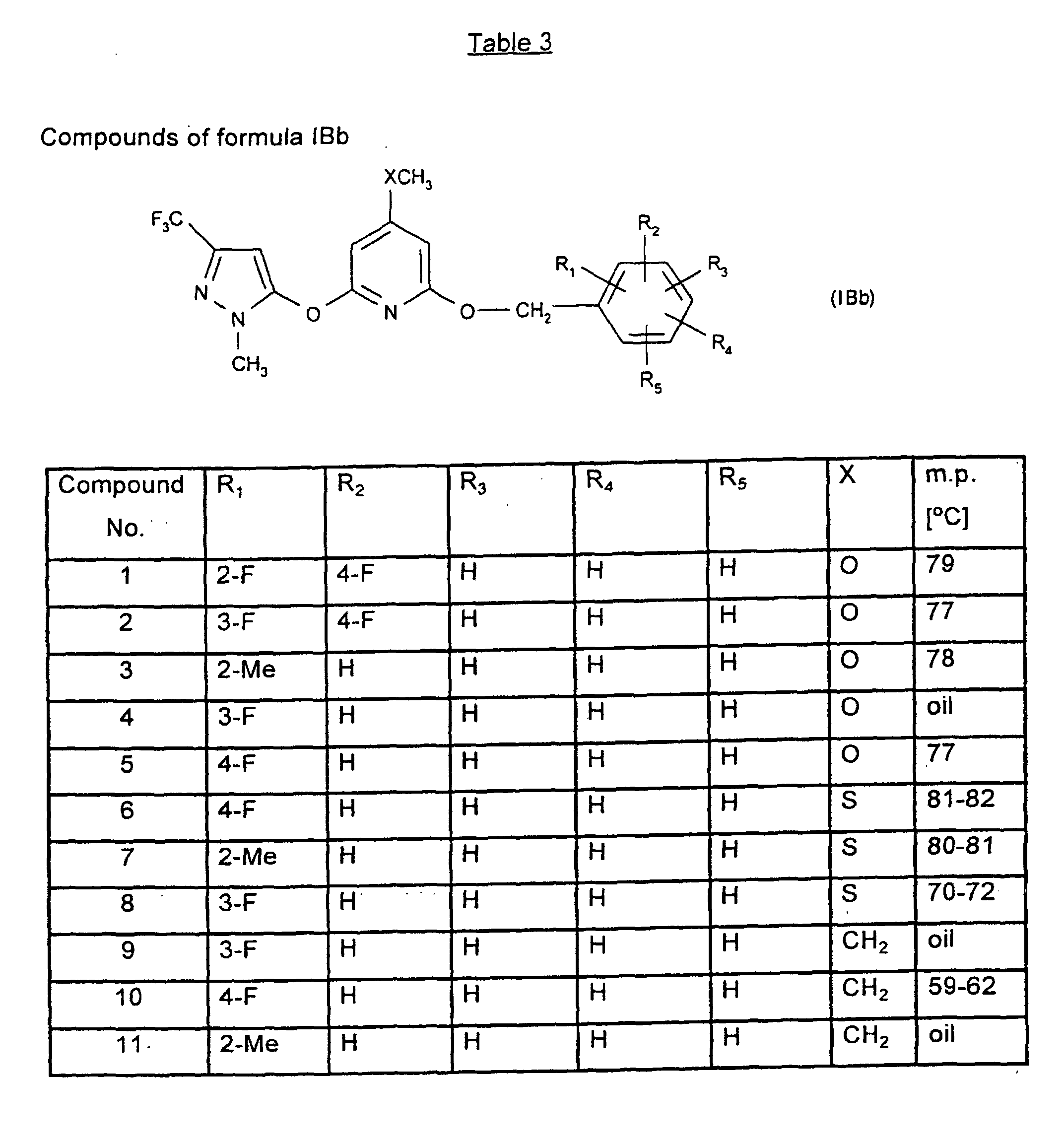

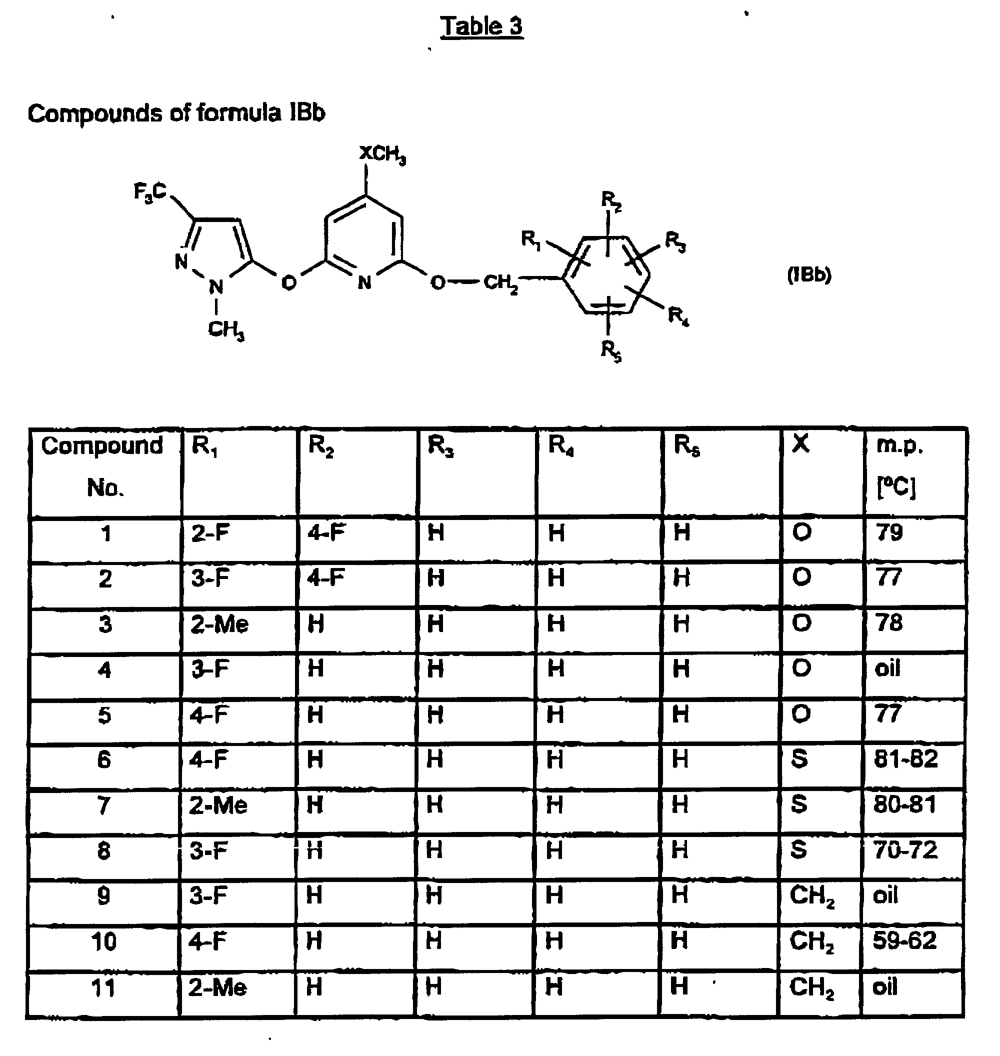

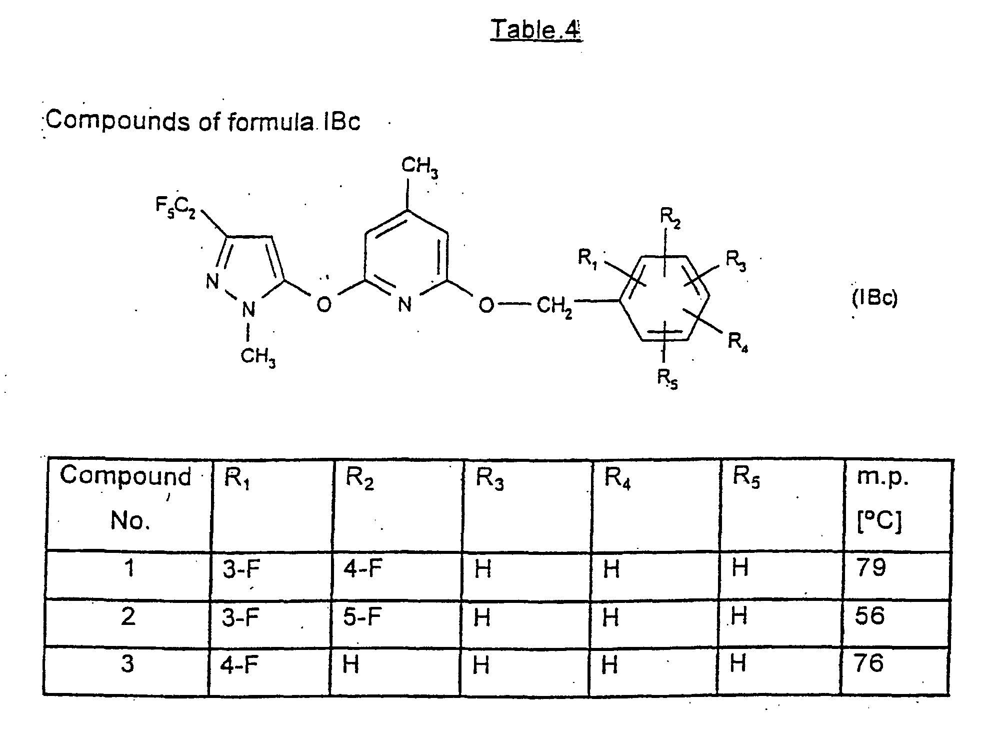

### Example 4

### Pre-emergence Herbicidal Evaluation of Test Compounds

The pre-emergence herbicidal activity of the compounds of the present invention is exemplified by the following test in which the seeds of a variety of monocotyledonous and dicotyledonous plants are seperately mixed with potting soil and planted on top of approximately one inch of soil in separate pots. After planting the pots are sprayed with the selected aqueous acetone solution containing test compound in sufficient quantity to provide the equivalent of about 0.0125 to 0.1 kg per hectare of test compound per pot. The treated pots are then placed on greenhouse benches, watered and cared for in accordance with conventional greenhouse procedures. From 2 to 4 weeks after treatment, the tests are terminated and each pot is examined and rated according to the rating system set forth below.

| Rating System | % Difference in Growth from the control |
|---|---|
| 0 - No effect | 0 |
| 1 - Trace effect | 1-5 |
| 2 - Slight effect | 6-15 |
| 3 - Moderate effect | 16-29 |
| 4 - Injury | 30-44 |
| 5 - Definite injury | 45-64 |
| 6 - Herbicidal effect | 65-79 |
| 7 - Good herbicidal effect | 80-90 |
| 8 - Aproaching complete kill | 91-99 |
| 9 - Complete kill | 100 |

| Plant Species Used | | |
|---|---|---|
| A = HORVW | Hordeum vulgare | winter barley |
| B = ZEAMX | Zea mays | maize |
| C = GLYMA | Glycine max | soyabeans |
| D = SETVI | Setaria viridis | green foxtail |
| E = ABUTH | Abutilon theophrasti | velvetlaef |
| F = IPOHE | Ipomoea hederacea | morning glory |
| G = MATIN | Matricaria inodora | mayweed |
| H = STEME | Stellaria media | chickweed |
| I = CHEAL | Chenopodium album | lambsquarters |
| J = AMBEL | Ambrosia artemiisifolia | ragweed |

The herbicidal proficiency of the active ingredients of the present invention is evident from the test results which are recorded in Table A below.

The following related known compounds were used as standards:
standard 1: 2-(1-methyl-3-trifluoromethylpyrazol-5-yloxy)-6-benzyloxy-4-methylpyridine;
standard 2: 2-(1-methyl-3-trifluoromethylpyrazol-5-yloxy)-6-(3-fluorophenoxy)-4-methylpyridine;
standard 3: 2-(1-methyl-3-trifluoromethylpyrazol-5-yloxy)-6-(2,4-difluorophenoxy)-4-methylpyridine.

**Table A:**

| Crop selectivity and weed control of compounds according to the invention bearing a 4-methoxy - substitution of the central pyridine ring in pre-emergence application. | | | | | | | | | | | |
|---|---|---|---|---|---|---|---|---|---|---|---|
| Compound | dose kg/ha | A | B | C | D | E | J | F | I | G | H |
| Table3/no.1 | 0.100 | 4 | 2 | 3 | 9 | 5 | 9 | 5 | 9 | 9 | 9 |
| | 0.025 | 1 | 1 | 2 | 8 | 3 | 7 | 3 | 8 | 8 | 7 |
| | 0.0125 | 1 | 1 | 2 | 6 | 3 | 6 | 2 | 8 | 8 | 6 |
| Table 3/no.2 | 0.100 | 4 | 2 | 3 | 9 | 5 | 8 | 4 | 9 | 9 | 8 |
| | 0.025 | 4 | 1 | 2 | 9 | 3 | 6 | 3 | 8 | 7 | 8 |
| | 0.0125 | 2 | 1 | 2 | 7 | 2 | 5 | 3 | 8 | 6 | 7 |
| Example 3 | 0.100 | 3 | 1 | 2 | 9 | 7 | 7 | 8 | 8 | 9 | 8 |
| | 0.025 | 1 | 1 | 2 | 5 | 4 | 7 | 5 | 7 | 8 | 6 |
| | 0.0125 | 1 | 0 | 1 | 4 | 4 | 7 | 4 | 6 | 8 | 6 |
| Standard 1* | 0.100 | 4 | 4 | 4 | 9 | 7 | 9 | 7 | 8 | 9 | 9 |
| | 0.025 | 3 | 2 | 4 | 9 | 7 | 8 | 7 | 9 | 8 | 9 |
| | 0.0125 | 3 | 1 | 2 | 9 | 4 | 7 | 4 | 8 | 8 | 7 |

| | | | | | | | | | | | |
|---|---|---|---|---|---|---|---|---|---|---|---|
| *2-(1-methyl-3-trifluoromethylpyrazol-5-yloxy)-6-benzyloxy-4-methylpyridine | | | | | | | | | | | |

The compounds of the invention were more selective in maize, soybeans and barley than standard 1. This was particularly true for the compound according to Example 3, which was much more selective than the standard.

### EXAMPLE 5

### Post-emergence Herbicidal Evaluation of Test Compounds

The post-emergence herbicidal activity of the compounds of the present invention is demonstrated by the following test, wherein a variety of monocotyledonous and dicotyledonous plants are treated with formulations prepared from solutions of the test compounds in acetone containing 0.4 % by weight of an alkylphenol/ethylene oxide condensate available under the trade mark TRITON X-155. These acetone solutions were diluted with water and the resulting formulations applied at dosage levels equivalent of about 0.0125 to 0.1 kg per hectare of test compound per pot. After spraying the plants are placed on greenhouse benches and are cared for in the usual manner, commensurate with conventional greenhouse practices. From 2 to 4 weeks after treatment, the seedling plants are examined and rated according to the rating system provided above. A rating 0 indicates growth as untreated control, while a rating 9 indicates death. The results of the test are set out in Table B below.

**Table B**

| Crop selectivity and weed control of compounds bearing a 4-methoxy - substitution of the central pyridine ring in post-emergence application | | | | | | | | | | | |
|---|---|---|---|---|---|---|---|---|---|---|---|
| Compound | dose kg/ha | A | B | C | D | E | J | F | I | G | H |
| Table3/no.1 | 0.100 | 3 | 2 | 6 | 8 | 9 | 5 | 9 | 8 | 8 | 4 |
| | 0.025 | 2 | 2 | 5 | 5 | 8 | 4 | 9 | 8 | 6 | 4 |
| | 0.0125 | 2 | 1 | 4 | 5 | 8 | 3 | 8 | 7 | 5 | 3 |
| Table 3/no.2 | 0.100 | 3 | 3 | 8 | 8 | 9 | 5 | 9 | 8 | 8 | 6 |
| | 0.025 | 3 | 2 | 7 | 6 | 7 | 5 | 9 | 8 | 6 | 5 |
| | 0.0125 | 2 | 2 | 5 | 4 | 7 | 3 | 7 | 8 | 5 | 4 |
| Example 3 | 0.100 | 3 | 2 | 6 | 6 | 9 | 7 | 9 | 8 | 8 | 5 |
| | 0.025 | 2 | 2 | 4 | 4 | 8 | 5 | 9 | 8 | 6 | 4 |
| | 0.0125 | 2 | 1 | 2 | 3 | 6 | 4 | 9 | 8 | 5 | 3 |
| Standard 1* | 0.100 | 4 | 4 | 8 | 7 | 9 | 6 | 9 | 8 | 7 | 6 |
| | 0.025 | 3 | 3 | 7 | 6 | 9 | 5 | 9 | 8 | 6 | 4 |
| | 0.0125 | 2 | 2 | 7 | 5 | 7 | 4 | 7 | 8 | 5 | 3 |

| | | | | | | | | | | | |
|---|---|---|---|---|---|---|---|---|---|---|---|
| *(2-(1-methyl-3-trifluoromethylpyrazol-5-yloxy)-6-benzyloxy-4-methylpyridine | | | | | | | | | | | |

In post-emergence application the compounds of the invention were clearly more selective in maize than the standard (see above). Two compounds of the present invention. Table 3/no. 1 and Example 3 were sufficiently selective in maize at the highest dose of 100 g/ha, while only 12.5 g/ha of the standard showed acceptable tolerance in maize. At the maize selective dose of 100 g/ha these compounds of the invention were clearly superior in activity over the standard. Furthermore, the compounds of the invention displayed also improved selectivity in barley when compared to the standard.

### EXAMPLE 6

Additional data on the pre-emergence activity of certain compounds of this invention and standard compounds is presented below in Tables C and D. The protocol is similar to that of Example 4, except that the dosages are those shown below. The rating system is a percentage of weeds eliminated versus untreated control; for example, table C shows that at 0.0150 Kg/ha the compound of the invention eliminated 60% of BROTE while the standard compound eliminated 35%.

**Table C**

| Efficacy of test compounds in pre-emergence application | | | | | | | | |
|---|---|---|---|---|---|---|---|---|
| Compound | dose kg/ha | ALOMY | LOLPE | APESV | BROTE | ABUTH | STEME | LAMPU |
| Table 2/no. 4 | 0.0600 | 100 | 95 | 100 | 90 | --- | 100 | 99 |
| | 0.0300 | 80 | 90 | 100 | 80 | 90 | 99 | 99 |
| | 0.0150 | 65 | 45 | 100 | 60 | 70 | 98 | 99 |
| | 0.0075 | 12 | 25 | 98 | 20 | 30 | 90 | 95 |
| Standard 1* | 0.0600 | 98 | 93 | 100 | 90 | --- | 98 | 99 |
| | 0.0300 | 70 | 65 | 98 | 80 | 65 | 97 | 99 |
| | 0.0150 | 55 | 20 | 98 | 35 | 55 | 92 | 98 |
| | 0.0075 | 15 | 10 | 97 | 20 | 35 | 55 | 97 |

| | | | | | | | | |
|---|---|---|---|---|---|---|---|---|
| *(2-(1-methyl-3-trifluoromethylpyrazol-5-yloxy)-6-benzyloxy-4-methylpyridine | | | | | | | | |

The compound of the invention showed good activity on both grasses and broad-leaved weeds at low doses. The activity on Alopecurus, Lolium, Stellaria and Abutilon was superior to that of the standard.

**Table D**

| Efficacy of the compounds of the invention in pre-emergence application | | | | | | | | | | |
|---|---|---|---|---|---|---|---|---|---|---|
| Compound | dose kg/ha | ALOMY | LOLPE | SETVI | APESV | BROTE | STEME | LAMPU | IPOHE | AMBEL |
| Table 1/no.3 | 0.0600 | 65 | 65 | 98 | 100 | 93 | 100 | 100 | 90 | 98 |
| | 0.0300 | 35 | 15 | 85 | 100 | 50 | 90 | 98 | 80 | 93 |
| | 0.0150 | 10 | 10 | 80 | 95 | 20 | 65 | 90 | 25 | 85 |
| | 0.0075 | 5 | 3 | 40 | 85 | 10 | 40 | 75 | 10 | 55 |
| Table 1/no.4 | 0.0600 | 85 | 90 | 97 | 100 | 93 | 100 | 100 | 75 | 100 |
| | 0.0300 | 75 | 85 | 90 | 100 | 75 | 100 | 100 | 60 | 100 |
| | 0.0150 | 45 | 55 | 85 | 100 | 55 | 92 | 95 | 35 | 85 |
| | 0.0075 | 10 | 25 | 65 | 96 | 20 | 85 | 90 | 30 | 60 |
| Table 1/no.5 | 0.0600 | 70 | 60 | 99 | 100 | 45 | 100 | 100 | 75 | 100 |
| | 0.0300 | 25 | 35 | 92 | 100 | 25 | 98 | 98 | 65 | 95 |
| | 0.0150 | 15 | 10 | 80 | 95 | 20 | 80 | 98 | 25 | 80 |
| | 0.0075 | 5 | 5 | --- | 75 | 10 | 60 | 95 | 15 | 75 |
| Table 1/no.1 | 0.0600 | 70 | 75 | 70 | 100 | 50 | 100 | 98 | 100 | 100 |
| | 0.0300 | 50 | 35 | 55 | 99 | 25 | 95 | 98 | 55 | 80 |
| | 0.0150 | 20 | 18 | 35 | 80 | 10 | 93 | 98 | 25 | 55 |
| | 0.0075 | 10 | 15 | 5 | 55 | 5 | 50 | 75 | 10 | 40 |
| Table 1/no.8 | 0.0600 | 85 | 75 | 100 | 100 | 55 | 100 | 98 | 60 | 97 |
| | 0.0300 | 60 | 45 | 90 | 98 | 30 | 70 | 98 | 20 | 85 |
| | 0.0150 | 10 | 25 | 85 | 92 | 18 | 55 | 90 | 10 | 50 |
| | 0.0075 | 5 | 5 | 25 | 65 | 5 | 20 | 45 | 5 | 15 |
| Standard 2* | 0.0600 | 70 | 75 | 70 | 100 | 50 | 100 | 98 | 60 | 97 |
| | 0.0300 | 50 | 35 | 55 | 99 | 25 | 80 | 95 | 15 | 55 |
| | 0.0150 | 20 | 18 | 35 | 80 | 10 | 50 | 93 | 5 | 40 |
| | 0.0075 | 10 | 15 | 5 | 55 | 5 | --- | 50 | 2 | 35 |

| | | | | | | | | | | |
|---|---|---|---|---|---|---|---|---|---|---|
| *2-(1-methyl-3-trifluoromethylpyrazol-5-yloxy)-6-(3-fluorophenoxy)-4-methylpyridine. | | | | | | | | | | |

The compounds of the invention showed clearly superior activity on both grasses and broad-leaved weeds over the standard. In particular, the compounds of Table 1 no.1, no. 4 and no. 8 exhibited good_activity on grasses such as Alopecurus, Lolium, Setaria, Apera and Bromus and furthermore on broad-leaved weeds such as Stellaria. Lamium, Ipomoea and Ambrosia.

### EXAMPLE 7

Post-emergence activity data is presented in Table E. The protocol is similar to that of Example 5, except that the dosages are those shown in Table E. Table E shows the percentage of weeds killed versus an untreated control.

**Table E**

| Efficacy of certain compounds according to the invention in post-emergence application | | | | | |
|---|---|---|---|---|---|
| Compound | dose [kg/ha] | MATIN | GALAP | LAMPU | STEME |
| Table 1/no.3 | 0.0300 | 65 | 50 | 92 | 45 |
| | 0.0150 | 55 | 35 | 90 | 35 |
| | 0.0075 | 35 | 35 | 85 | 30 |
| Table 1/no. 4 | 0.0300 | 55 | 70 | 90 | 55 |
| | .0150 | 55 | 70 | 85 | 35 |
| | 0.0075 | 25 | 45 | 75 | 30 |
| Table 1/no. 5 | 0.0300 | 85 | 45 | 92 | 40 |
| | 0.0150 | 75 - | 25 | 92 | 30 |
| | 0.0075 | 35 | 12 | 80 | 20 |
| standard 3* | 0.0300 | 30 | 35 | 80 | 35 |
| | 0.0150 | 25 | 25 | 60 | 25 |
| | 0.0075 | 12 | 15 | 50 | 20 |

| | | | | | |
|---|---|---|---|---|---|
| *2-(1-methyl-3-trifluoromethylpyrazol-5-yloxy)-6-(2,4-difluorophenoxy)- 4-methylpyridine. | | | | | |

The compounds of the invention showed clearly superior activity over the standard on broad-leaved weeds.

## Claims

1. A compound of formula IA, 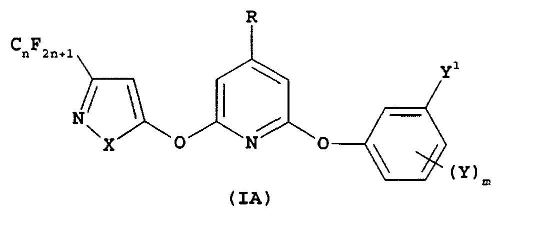 in which
R represents a methyl, ethyl, methoxy, ethoxy, methylthio or ethylthio group or a fluorine, chlorine or bromine atom;
X represents -(N(CH₃)-;
Y¹ represents a fluorine or chlorine atom ora methyl, trifluoromethyl or nitro group;
Y each independently represents a fluorine or chlorine atom or a methyl, trifluoromethyl or nitro group;
m is an integer from 0 to 2; and
n is 1 or 2;
with the proviso that m is 1 or 2, if Y¹ represents a fluorine atom;
and the agronomically acceptable salts thereof.

2. A compound of formula IB, 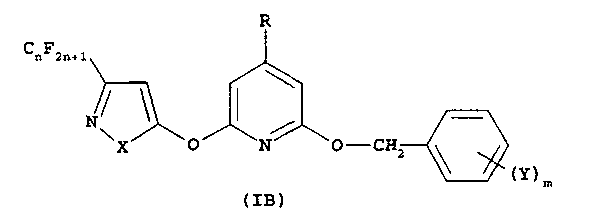 in which
R represents a methyl, ethyl, methoxy, ethoxy, methylthio or ethylthio group or a fluorine, chlorine or bromine atom;
X represents -(N(CH₃)-;
Y each independently represents a fluorine or chlorine atom or a methyl, trifluoromethyl or nitro group;
m is an integer from 1 to 3; and
n is 1 or 2;
and the agronomically acceptable salts thereof.

3. A compound as claimed in claims 1 or 2 wherein R is methyl or methoxy.

4. A compound as claimed in any of the claims 1 and 3, wherein
n is 1; and the group 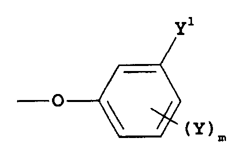 is selected from the group consisting of 3,4-difluorophenyl, 3,5-difluorophenyl, 3-chloro-4-fluorophenyl, 3-trifluoromethyl-4-fluorophenyl and 3-trifluoromethyl-4-chlorophenyl.

5. A compound as claimed in any of the claims 2 to 3, wherein
n is 1; and the group 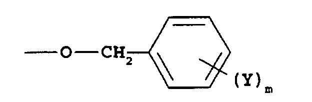 is selected from the group consisting of fluorobenzyl, chlorobenzyl, methylbenzyl, difluorobenzyl, dichlorobenzyl, trifluoromethylbenzyl, fluoro-trifluoromethylbenzyl and 3-trifluoromethyl-4-nitrobenzyl.

6. A compound as claimed in any of the claims 1 to 5 selected from the group consisting of
2-(1-methyl-3-trifluoromethylpyrazol-5-yloxy)-6-(2-methylbenzyloxy)-4-methoxypyridine;
2-(1-methyl-3-trifluoromethylpyrazol-5-yloxy)-6-(2,4-difluorobenzyloxy)-4-methoxypyridine;
2-(1-methyl-3-trifluoromethylpyrazol-5-yloxy)-6-(3,4-difluorobenzyloxy)-4-methoxypyridine;
2-(1-methyl-3-trifluoromethylpyrazol-5-yloxy)-6-(4-fluorobenzyloxy)-4-methylpyridine;
2-(1-methyl-3-trifluoromethylpyrazol-5-yloxy)-6-(3,4-difluorophenoxy)-4-methylpyridine;
2-(1-methyl-3-trifluoromethylpyrazol-5-yloxy)-6-(3-chloro-4-fluorophenoxy)-4-methylpyridine;
2-(1-methyl-3-trifluoromethylpyrazol-5-yloxy)-6-(4-fluoro-3-trifluoromethylphenoxy)-4-methylpyridine;
2-(1-methyl-3-trifluoromethylpyrazol-5-yloxy)-6-(4-chloro-3-trifluoromethylphenoxy)-4-methylpyridine;
2-(1-methyl-3-trifluoromethylpyrazol-5-yloxy)-6-(3-trifluoromethylphenoxy)-4-methylpyridine; and.
2-(1-methyl-3-trifluoromethylpyrazol-5-yloxy)-6-(3,5-difluorophenoxy)-4-methyl-pyridine.

7. A herbicidal composition comprising as active ingredient a compound as claimed in any claims 1 to 6, and a carrier.

8. A herbicidal composition according to claim 7 further comprising a surface-active agent.

9. A method for combating undesired plants, which method comprises treating a locus with an effective amount of a compound as claimed in any of the claims 1 to 6.

10. A method for selectively combating undesired plants in barley, maize or soybeans which comprises contacting said undesired plants with an effective amount of a compound as claimed in any of the claims 1 to 6.

## Patentansprüche

1. Verbindung der Formel IA, 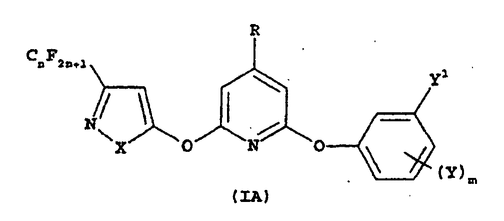 in der
R eine Methyl-, Ethyl-, Methoxy-, Ethoxy-, Methylthio- oder Ethylthiogruppe oder ein Fluor-, Chlor- oder Bromatom bedeutet,
X -(N(CH₃)- bedeutet,
Y¹ ein Fluor- oder Chloratom oder eine Methyl-, Trifluormethyl- oder Nitrogruppe bedeutet,
die einzelnen Y unabhängig voneinander ein Fluor- oder Chloratom oder eine Methyl-, Trifluormethyl- oder Nitrogruppe bedeuten,
m eine ganze Zahl von 0 bis 2 ist und
n 1 oder 2 ist,
mit der Maßgabe, daß, wenn Y¹ ein Fluoratom bedeutet, m 1 oder 2 ist,
sowie deren landwirtschaftlich verträgliche Salze.

2. Verbindung der Formel IB, 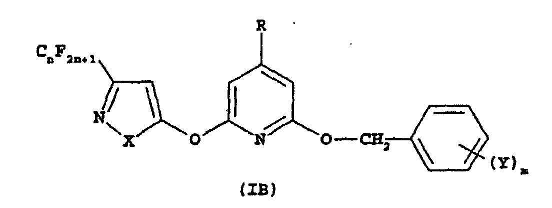 in der
R eine Methyl-, Ethyl-, Methoxy-, Ethoxy-, Methylthio- oder Ethylthiogruppe oder ein Fluor-, Chlor- oder Bromatom bedeutet,
X -(N(CH₃)- bedeutet,
die einzelnen Y unabhängig voneinander ein Fluor- oder Chloratom oder eine Methyl-, Trifluormethyl- oder Nitrogruppe bedeuten,
m eine ganze Zahl von 1 bis 3 ist und
n 1 oder 2 ist,
sowie deren landwirtschaftlich verträgliche Salze.

3. Verbindung nach Anspruch 1 oder 2, worin R Methyl oder Methoxy bedeutet.

4. Verbindung nach einem der Ansprüche 1 und 3, worin
n 1 ist und die Gruppe 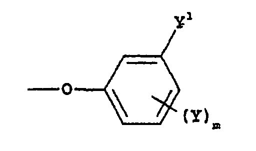
aus der Gruppe 3,4-Difluorphenyl, 3,5-Difluorphenyl, 3-Chlor-4-fluorphenyl, 3-Trifluormethyl-4-fluorphenyl und 3-Trifluormethyl-4-chlorphenyl stammt.

5. Verbindung nach einem der Ansprüche 2 bis 3, wobei
n 1 ist und die Gruppe 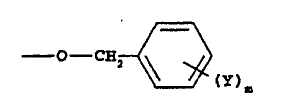
aus der Gruppe Fluorbenzyl, Chlorbenzyl, Methylbenzyl, Difluorbenzyl, Dichlorbenzyl, Trifluormethylbenzyl, Fluortrifluormethylbenzyl und 3-Trifluormethyl-4-nitrobenzyl stammt.

6. Verbindung nach einem der Ansprüche 1 bis 5 aus der Gruppe
2-(1-Methyl-3-trifluormethylpyrazol-5-yloxy)-6-(2-methylbenzyloxy) -4-methoxypyridin,
2-(1-Methyl-3-trifluormethylpyrazol-5-yloxy)-6-(2,4-difluorbenzyloxy)-4-methoxypyridin,
2-(1-Methyl-3-trifluormethylpyrazol-5-yloxy)-6-(3,4-difluorbenzyloxy)-4-methoxypyridin,
2-(1-Methyl-3-trifluormethylpyrazol-5-yloxy)-6-(4-fluorbenzyloxy)-4-methylpyridin,
2-(1-Methyl-3-trifluormethylpyrazol-5-yloxy)-6-(3,4-difluorphenoxy)-4-methylpyridin,
2-(1-Methyl-3-trifluormethylpyrazol-5-yloxy)-6-(3-chlor-4-fluorphenoxy)-4-methylpyridin,
2-(1-Methyl-3-trifluormethylpyrazol-5-yloxy)-6-(4-fluor-3-trifluormethylphenoxy)-4-methylpyridin,
2-(1-Methyl-3-trifluormethylpyrazol-5-yloxy)-6-(4-chlor-3-trifluormethylphenoxy)-4-methylpyridin,
2-(1-Methyl-3-trifluormethylpyrazol-5-yloxy)-6-(3-trifluormethylphenoxy)-4-methylpyridin
und
2-(1-Methyl-3-trifluormethylpyrazol-5-yloxy)-6-(3,5-difluorphenoxy)-4-methylpyridin.

7. Herbizide Zusammensetzung, die eine Verbindung nach einem der Ansprüche 1 bis 6 als Wirkstoff sowie einen Träger enthält.

8. Herbizide Zusammensetzung nach Anspruch 7, die weiterhin ein oberflächenaktives Mittel enthält.

9. Verfahren zur Bekämpfung von unerwünschten Pflanzen, **dadurch gekennzeichnet, daß** man einen Ort mit einer wirksamen Menge einer Verbindung nach einem der Ansprüche 1 bis 6 behandelt.

10. Verfahren zur selektiven Bekämpfung von unerwünschten Pflanzen in Gerste, Mais oder Sojabohnen, **dadurch gekennzeichnet, daß** man die unerwünschten Pflanzen mit einer wirksamen Menge einer Verbindung nach einem der Ansprüche 1 bis 6 in Kontakt bringt.

## Revendications

1. Composé de formule IA, 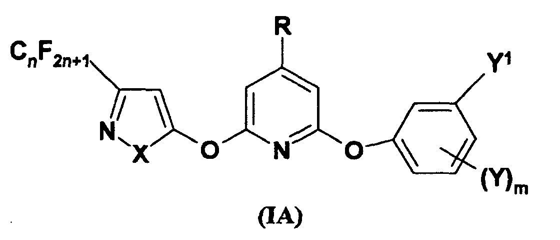 dans laquelle
R représente un groupe méthyle, éthyle, méthoxy, éthoxy, méthylthio ou éthylthio, ou un atome de fluor, de chlore, ou de brome;
X représente -N(CH₃)-;
Y¹ représente un atome de fluor ou de chlore ou un groupe méthyltrifluorométhyle ou nitro;
Y représente chacun indépendamment un atome de fluor ou de chlore ou un groupe méthyle, trifluorométhyle ou nitro;
m est un nombre entier de 0 à 2; et
n vaut 1 ou 2;
avec la condition que m vaut 1 ou 2, si Y¹ représente un atome de fluor;
et les sels de celui-ci, acceptables sur le plan agronomique.

2. Composé de formule IB, 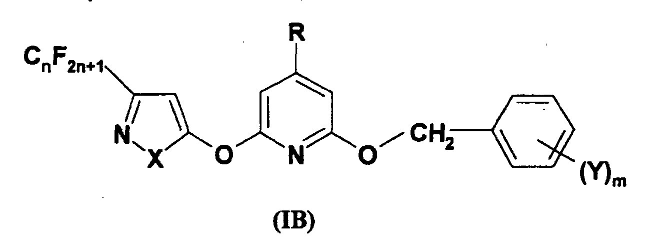 dans laquelle
R représente un groupe méthyle, éthyle, méthoxy, éthoxy, méthylthio ou éthylthio, ou un atome de fluor, de chlore, ou de brome;
X représente-N(CH₃)-;
Y représente chacun indépendamment un atome de fluor ou de chlore ou un groupe méthyle, trifluorométhyle, ou nitro;
m est un nombre entier de 1 à 3; et
n vaut 1 ou 2;
et les sels de celui-ci, acceptables sur le plan agronomique.

3. Composé selon les revendications 1 ou 2, dans lequel R est un groupe méthyle ou méthoxy.

4. Composé selon l'une quelconque des revendications 1 et 3, dans lequel n vaut 1; et le groupe 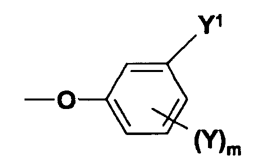 est choisi dans le groupe formé par un groupe 3,4-difluorophényle, 3,5-difluorophényle, 3-chloro-4-fluorophényle, 3-trifluorométhyl-4-fluorophényle et 3-trifluorométhyl-4-chlorophényle.

5. Composé selon l'une quelconque des revendications 2 à 3, dans lequel n vaut 1 et le groupe 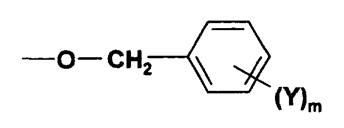 est choisi dans le groupe formé par un groupe fluorobenzyle, chlorobenzyle, méthylbenzyle, difluorobenzyle, dichlorobenzyle, trifluorométhylbenzyle, fluoro-trifluorométhylbenzyle, et 3-trifluorométhyl-4-nitrobenzyle.

6. Composé selon l'une quelconque des revendications 1 à 5, choisi dans le groupe formé par
la 2-(1-méthyl-3-trifluorométhylpyrazol-5-yloxy)-6-(2-méthylbenzyloxy)-4-méthoxypyridine;
la 2-(1-méthyl-3-trifluorométhylpyrazol-5-yloxy)-6-(2,4-difluorobenzyloxy)-4-méthoxypyridine;
la 2-(1-méthyl-3-trifluorométhylpyrazol-5-yloxy)-6-(3,4-difluorobenzyloxy)-4-méthoxypyridine;
la 2-(1-méthyl-3-trifluorométhylpyrazol-5-yloxy)-6-(4-fluorobenzyloxy)-4-méthylpyridine;
la 2-(1-méthyl-3-trifluorométhylpyrazol-5-yloxy)-6-(3,4-difluorophénoxy)-4-méthylpyridine;
la 2-(1-méthyl-3-trifluorométhylpyrazol-5-yloxy)-6-(3-chloro-4-fluorophénoxy)-4-méthylpyridine;
la 2-(1-méthyl-3-trifluorométhylpyrazol-5-yloxy)-6-(4-fluoro-3-trifluorométhylphénoxy)-4-méthylpyridine;
la 2-(1-méthyl-3-trifluorométhylpyrazol-5-yloxy)-6-(4-chloro-3-trifluorométhylphénoxy)-4-méthylpyridine;
la 2-(1-méthyl-3-trifluorométhylpyrazol-5-yloxy)-6-(3-trifluorométhylphénoxy)-4-méthylpyridine; et
la 2-(1-méthyl-3-trifluorométhylpyrazol-5-yloxy)-6-(3,5-difluorophénoxy)-4-méthylpyridine.

7. Composition herbicide comprenant en tant qu'ingrédient actif un composé selon l'une quelconque des revendications 1 à 6, et un véhicule.

8. Composition herbicide selon la revendication 7, comprenant de plus un agent tensio-actif.

9. Procédé pour lutter contre des plantes indésirables, lequel procédé comprend le traitement d'un lieu par une quantité efficace d'un composé selon l'une quelconque des revendications 1 à 6.

10. Procédé pour lutter de façon sélective contre des plantes indésirables dans l'orge, le maïs, ou le soja, qui comprend la mise en contact desdites plantes indésirables avec une quantité efficace d'un composé selon l'une quelconque des revendications 1 à 6.
